# EUROPEAN PATENT APPLICATION

(11) **EP 4 599 764 A1**
(43) Date of publication of application: **13.08.2025**
(21) Application number: 23920091.8
(22) Date of filing: 24.07.2023
(51) Int. Cl.: A61B 5/11, A61B 5/00

(54) **PORTABLE ELECTRONIC DEVICE FOR STEP DETECTION AND OPERATING METHOD THEREOF**

(30) Priority: 30.01.2023 KR 20230011578; 28.04.2023 KR 20230056395
(71) Applicant: Samsung Electronics Co., Ltd., Suwon-si, Gyeonggi-do 16677 (KR)
(72) Inventor: BAE, Hyunjin, Suwon-si, Gyeonggi-do 16677 (KR); PARK, Sunyoung, Suwon-si, Gyeonggi-do 16677 (KR); KANG, Jeonggwan, Suwon-si, Gyeonggi-do 16677 (KR); SHIN, Seunghyuck, Suwon-si, Gyeonggi-do 16677 (KR)
(74) Representative: HGF
(86) International application number: PCT/KR2023/010646
(87) International publication number: WO 2024/162541

(57) **Abstract**

Provided is a portable electronic device including: a wireless communication circuit; a sensing circuit; a processor connected to the wireless communication circuit and the sensing circuit; and a memory operatively connected to the processor. The memory may store instructions which cause, when executed, the processor to: receive first information indicating that an exercise other than walking and running is being performed, from a wearable device through the wireless communication circuit; stop a step detection operation using the sensing circuit, on the basis of the reception of the first information; receive second information indicating that the exercise is ended, from the wearable device through the wireless communication circuit, while the detection operation is stopped; and resume the detection operation, on the basis of the reception of the second information.

## Description

### [Technical Field]

An embodiment of the present disclosure relates to a portable electronic device for detecting a user's step using an inertial sensor and a method for operating the same.

### [Background Art]

A portable electronic device may measure an amount of change in acceleration according to user's movement using data generated by an inertial sensor, analyze a pattern of a change in acceleration, and determine whether the analyzed pattern is a pattern corresponding to a shape of a step. The portable electronic device may detect a step when a signal characteristic value of a determined pattern satisfies a designated condition.

The above-described information is provided as the related art for the purpose of helping to understand the present disclosure. None of the above-described contents raises any claim or determination regarding the applicability of prior art related to the present disclosure.

### [Disclosure of Invention]

### [Technical Problem]

Errors may occur when a portable electronic device counts steps while a user is carrying a portable electronic device and an exercise (e.g., a bicycle, a kickboard, or a motorcycle) other than walking and running is performed. The portable electronic device can solve errors occurring when counting steps using location data acquired from a GPS sensor while a user is performing the exercise other than walking and running.

In an embodiment of the present disclosure, the portable electronic device can further improve the accuracy of step detection without driving the GPS sensor (i.e., without consuming power for driving the GPS sensor).

The technical problems to be achieved by the present disclosure are not limited to the above-mentioned objects, and other technical problems that are not mentioned may be obviously understood by those skilled in the art to which the present invention pertains from the following description.

### [Solution to Problem]

According to an embodiment, a portable electronic device includes: a wireless communication circuit; a sensing circuit; a processor connected to the wireless communication circuit and the sensing circuit; and a memory operatively connected to the processor. The memory may store instructions which cause, when executed, the processor to receive first information indicating that an exercise other than walking and running is being performed, from a wearable device through the wireless communication circuit. The instructions may cause the processor to stop a step detection operation using the sensing circuit based on the reception of the first information. The instructions may cause the processor to receive second information indicating that the exercise is ended, from the wearable device through the wireless communication circuit, while the detection operation is stopped. The instructions may cause the processor to resume the detection operation based on the reception of the second information.

According to an embodiment, a portable electronic device includes: a touch-sensitive display; an input device; a sensing circuit; a processor connected to the display, the input device, and the sensing circuit; and a memory operatively connected to the processor. The memory may store instructions which cause, when executed, the processor to receive first information indicating that an exercise other than walking and running is being performed, from the display or the input device. The instructions may cause the processor to stop a step detection operation using the sensing circuit based on the reception of the first information. The instructions may cause the processor to receive second information indicating that the exercise is ended, from the display or the input device, while the detection operation is stopped. The instructions may cause the processor to resume the detection operation based on the reception of the second information.

According to an embodiment, a method of operating a portable electronic device may include receiving first information indicating that an exercise other than walking and running is being performed, from a wearable device through a wireless communication circuit of the portable electronic device. The method may include stopping a step detection operation using the sensing circuit of the portable electronic device based on the reception of the first information. The method may include receiving second information indicating that the exercise is ended, from the wearable device through the wireless communication circuit, while the detection operation is stopped. The method may include resuming the detection operation based on the reception of the second information.

### [Advantageous Effects of Invention]

According to an embodiment of the present disclosure, a portable electronic device can improve the accuracy of step detection without a GPS sensor. In addition, various effects that are directly or indirectly understood through the present document may be provided.

### [Brief Description of Drawings]

FIG. 1 is a block diagram of an electronic device in a network environment according to various embodiments.
FIG. 2 illustrates a configuration of a system for detecting a step based on exercise information according to an embodiment.
FIG. 3 is a diagram illustrating an example of a user interface (UI) screen displayed on a wearable device.
FIG. 4 is a diagram illustrating an example of the UI screen displayed on a portable electronic device.
FIG. 5 is a flowchart for describing operations of a second processor for determining reliability according to an embodiment.
FIG. 6 is a flowchart for describing the operations of the second processor for controlling step detection based on reliability according to an embodiment.
FIG. 7 is a diagram for describing operations of controlling step detection based on a user's determination on a start and end of the bicycle exercise in the system of FIG. 2 according to an embodiment.
FIG. 8 is a diagram for describing the operations of controlling step detection based on the recognition of the start and end of the bicycle exercise in the system of FIG. 2 according to an embodiment.
FIG. 9 is a flowchart for describing the operations performed by the second processor of the portable electronic device based on information received from the wearable device according to an embodiment.
FIG. 10 is a flowchart for describing the operations performed by the second processor of the portable electronic device based on user input according to an embodiment.
FIG. 11 is a flowchart for describing the operations performed based on information received from the wearable device according to an embodiment.
FIG. 12 is a flowchart for describing the operations performed based on the information received from the wearable device according to an embodiment.

Hereinafter, embodiments of the present disclosure will be described in detail with reference to the drawings so that those skilled in the art to which the present disclosure pertains may easily practice the disclosure. However, the disclosure may be implemented in various different forms, and is not limited to embodiments described herein. In connection with the description of the drawings, the same or similar reference numerals may be used for the same or similar components. In addition, in the drawings and related descriptions, descriptions of well-known functions and configurations may be omitted for clarity and conciseness.

### [Mode for the Invention]

Hereinafter, embodiments of the present disclosure will be described in detail with reference to the drawings so that those skilled in the art to which the present disclosure pertains may easily practice the disclosure. However, the disclosure may be implemented in various different forms, and is not limited to embodiments described herein. In connection with the description of the drawings, the same or similar reference numerals may be used for the same or similar components. In addition, in the drawings and related descriptions, descriptions of well-known functions and configurations may be omitted for clarity and conciseness.

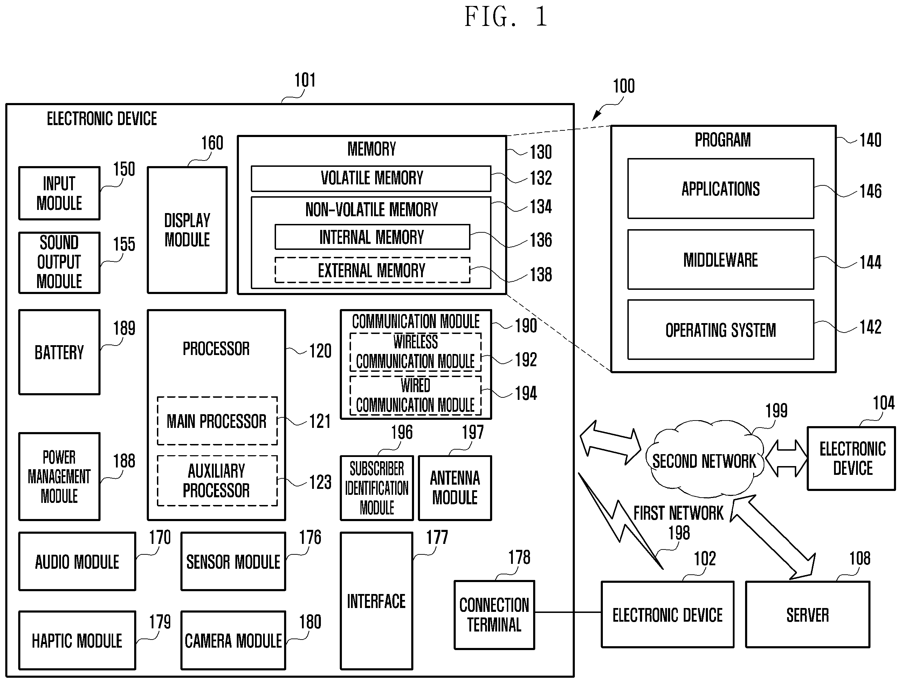

Fig. 1 is a block diagram illustrating an electronic device 101 in a network environment 100 according to various embodiments. Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a **HDMI** connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

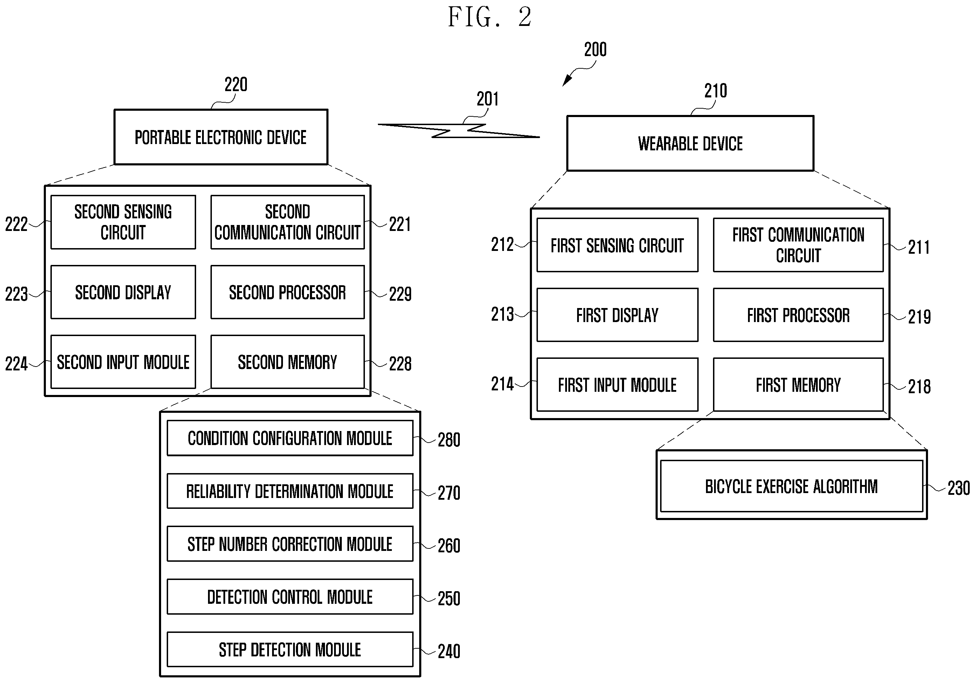

FIG. 2 illustrates a configuration of a system 200 for detecting a step based on exercise information according to an embodiment. Referring to FIG. 2, the system 200 may include a wearable device 210 and a portable electronic device 220.

The wearable device 210 (e.g., a smart watch) may include a first communication circuit 211, a first sensing circuit 212, a first display 213, a first input module 214, a first memory 218, and a first processor 219. The above-described components of the wearable device 210 may be operatively or electrically connected to each other. The first communication circuit 211, the first sensing circuit 212, the first display 213, the first input module 214, the first memory 218, and the first processor 219 are implemented to be substantially the same as the wireless communication module 192, the sensor module 176, the display module 160, the input module 150, the memory 130, and the processor 120 of FIG. 1, respectively, and thus, may perform the same functions.

The first communication circuit 211 may include a short-range wireless communication circuit configured to establish a wireless communication channel 201 between the wearable device 210 and the portable electronic device 220 and perform data communication between the two devices 210 and 220 through the established wireless communication channel 201. For example, the first communication circuit 211 may include a Bluetooth communication circuit. Additionally, the first communication circuit 211 may include a WiFi communication circuit. The first processor 219 may perform data communication with the portable electronic device 220 through the short-range wireless communication channel (e.g., a Bluetooth communication channel or a WiFi communication channel) established by the first communication circuit 211.

The first sensing circuit 212 is configured to recognize a bicycle exercise and generate data used to monitor the process of the bicycle exercise, and may provide the generated data to the first processor 219. For example, the first sensing circuit 212 may include an inertia measurement unit (IMU) that is configured to include an acceleration sensor, a gyroscope, and a magnetometer.

The bicycle exercise algorithm 230 is composed of instructions, and may be stored in the first memory 218 and executed by the first processor 219. The first processor 219 may store data generated by executing the bicycle exercise algorithm 230 in the first memory 218.

The bicycle exercise algorithm 230 may be configured to identify whether the bicycle is in operation (in other words, while a user is performing the bicycle exercise) using data received from the first sensing circuit 212. For example, the bicycle exercise algorithm 230 may monitor physical quantities (e.g., acceleration, angular velocity, magnetic field) from the data received from the first sensing circuit 212. When the bicycle is in operation, the physical quantities acquired through monitoring may satisfy predetermined bicycle exercise patterns. When the bicycle is stopped, the acquired physical quantities may not satisfy the bicycle exercise patterns. The bicycle exercise algorithm 230 may recognize that the user is performing the bicycle exercise when the acquired physical quantities satisfy the bicycle exercise patterns.

According to an embodiment, the bicycle exercise algorithm 230 may be configured to transmit, to the portable electronic device 220 through the first communication circuit 211, a first notification message that notifies the portable electronic device 220 that the bicycle exercise is started based on recognizing that the bicycle exercise is started. For example, the bicycle exercise algorithm 230 may recognize that the bicycle exercise is started when the acquired physical quantities satisfy the bicycle exercise patterns, and transmit the first notification message to the portable electronic device 220 through the first communication circuit 211 accordingly. For another example, the bicycle exercise algorithm 230 may recognize that the bicycle exercise is started when the physical quantities satisfying the bicycle exercise patterns are repeatedly acquired for a designated time (e.g., 3 minutes). Based on recognizing that the bicycle exercise is started, the bicycle exercise algorithm 230 may transmit the first notification message to the portable electronic device 220 through the first communication circuit 211. The bicycle exercise algorithm 230 may include information indicating the time point at which the bicycle exercise patterns is first satisfied in a first notification message and transmit the first notification message to the portable electronic device 220. The bicycle exercise algorithm 230 may stop transmitting the scheduled first notification message based on the physical quantity that do not satisfy the bicycle exercise patterns being acquired before the designated time has elapsed. In the following description, the time point at which the bicycle exercise patterns are first satisfied is referred to as a 'first time point', and the time point at which the first notification message is transmitted after the first time point is referred to as a 'second time point'.

According to an embodiment, the bicycle exercise algorithm 230 may be configured to repeatedly transmit, to the portable electronic device 220 through the first communication circuit 211, every designated time a second notification message that enables the portable electronic device 220 to recognize that the bicycle exercise is being maintained while the bicycle exercise is being maintained after the first notification message is transmitted.

According to an embodiment, the bicycle exercise algorithm 230 may be configured to monitor the process of the bicycle exercise using the data received from the first sensing circuit 212 and record the monitored result in the first memory 218. For example, the bicycle exercise algorithm 230 may measure the time (hereinafter, exercise duration) when the physical quantities acquired through the monitoring are recognized as satisfying the bicycle exercise patterns and the time (hereinafter, rest duration) when the physical quantities are recognized as not satisfying the bicycle exercise patterns based on the data received from the first sensing circuit 212, and store the measured results in the first memory 218.

According to an embodiment, the bicycle exercise algorithm 230 may be configured to repeatedly transmit bicycle exercise information related to the exercise duration per unit time to the portable electronic device 220 through the first communication circuit 211. For example, the bicycle exercise algorithm 230 may measure the exercise duration and the rest duration during a predetermined duration of time (e.g., 10 minutes), and provide the bicycle exercise information including the measured exercise duration and rest duration to the portable electronic device 220 every time the unit time elapses. For another example, the bicycle exercise algorithm 230 may calculate a ratio of the exercise duration to the rest duration during a predetermined duration of time, and provide bicycle exercise information including the calculated ratio to the portable electronic device 220 every time the unit time elapses. For another example, the bicycle exercise algorithm 230 may calculate a multiple value that indicates how many times the exercise duration is greater than the rest duration during a predetermined duration of time, and provide the bicycle exercise information including the calculated multiple value to the portable electronic device 220 every time the unit time elapses.

For example, a transmission time point of the bicycle exercise information may be substantially the same as that of the second notification message. The bicycle exercise algorithm 230 may include the bicycle exercise information in the second notification message and provide the second notification message to the portable electronic device 220.

According to an embodiment, the bicycle exercise algorithm 230 may be configured to recognize that the bicycle exercise is ended based on the rest duration during which the bicycle is recognized as not being operated exceeding a designated threshold, and end the monitoring and recording accordingly. **In** addition, the bicycle exercise algorithm 230 may be configured to transmit, to the portable electronic device 220 through the first communication circuit 211, a third notification message that notifies the portable electronic device 220 that the bicycle exercise is ended based on the rest duration exceeding the specified threshold.

The first communication circuit 211 may further include, for example, a GNSS communication circuit as a communication circuit for identifying a geographical location of the wearable device 210. The bicycle exercise algorithm 230 may additionally use data acquired from the GNSS communication circuit in addition to data acquired from the first sensing circuit 212 in performing the recognition and monitoring of bicycle exercise.

The first sensing circuit 212 may further include a heart rate detection sensor. The bicycle exercise algorithm 230 may further use data received from the heart rate detection sensor to perform the recognition and monitoring of the bicycle exercise. For example, the bicycle exercise algorithm 230 may acquire vibration patterns (e.g., vibration frequency, amplitude, intensity) from data received from an inertial sensor, acquire a speed using data received from the GNSS communication circuit, and acquire a heart rate from data received from a heart rate detection sensor. The bicycle exercise algorithm 230 may recognize that the user is performing the bicycle exercise based on whether the acquired information satisfies the predetermined bicycle exercise patterns.

According to an embodiment, the first display 213 and/or the first input module 214 may be used as input devices that notify the portable electronic device 220 of the start and end of the bicycle exercise.

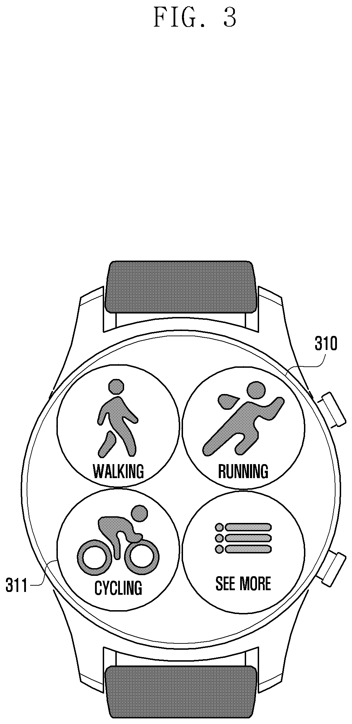

FIG. 3 is a diagram illustrating an example of a user interface (UI) screen displayed on the wearable device 210. Referring to FIG. 3, the first processor 219 may provide, to a user through the first display 213, a first UI screen 310 that enables the user to determine the start of the bicycle exercise. The bicycle exercise algorithm 230 may receive a user input indicating the start of the bicycle exercise through the first UI screen 310 displayed on the first display 213. For example, when a user touches a display object 311 (e.g., an icon) that is displayed as 'cycling' on the first UI screen 310, the bicycle exercise algorithm 230 may recognize that a user input corresponding to a touch is transmitted from the first display 213 to the first processor 219.

Referring to FIG. 2, the first processor 219 may provide, to a user through the first display 213, a second UI screen (not illustrated) that enables the user to determine the end of the bicycle exercise. The bicycle exercise algorithm 230 may receive a user input indicating the end of the bicycle exercise through the second UI screen displayed on the first display 213.

The bicycle exercise algorithm 230 may also receive the user input indicating the start or end of the bicycle exercise through the first input module 214 (e.g., a microphone or a physical button).

According to an embodiment, the bicycle exercise algorithm 230 may be configured to transmit the first notification message to the portable electronic device 220 based on the reception of the user input indicating the start of the bicycle exercise. The bicycle exercise algorithm 230 may be configured to transmit the third notification message to the portable electronic device 220 based on the reception of the user input indicating the end of the bicycle exercise.

According to an embodiment, the wearable device 210 may include various exercise algorithms configured to perform substantially the same function as the bicycle exercise algorithm 230 described above, except that the type of exercises is different. For example, the wearable device may include an exercise algorithm configured to identify whether the user is riding a kickboard or a motorcycle by using the data received from the sensing circuit (e.g., the first sensing circuit 212 of FIG. 2). In addition, the wearable device 210 may provide the UI screen to the user through the first display 213 that may allow the user to determine the start and end of the kickboard or motorcycle exercise.

The portable electronic device 220 (e.g., a smart phone) may include a second communication circuit 221, a second sensing circuit 222, a second display 223, a second input module 224, a second memory 228, and a second processor 229. The above-described components of the portable electronic device 220 may be operatively or electrically connected to each other. The second communication circuit 221, the second sensing circuit 222, the second display 223, the second input module 224, the second memory 228, and the second processor 229 are implemented to be substantially the same as the wireless communication module 192, the sensor module 176, the display module 160, the input module 150, the memory 130, and the processor 120 of FIG. 1, respectively, and thus, may perform the same functions.

The second communication circuit 221 may include the short-range wireless communication circuit configured to establish the wireless communication channel 201 between the wearable device 210 and the portable electronic device 220 and perform data communication between the two devices 210 and 220 through the established wireless communication channel 201. For example, the second communication circuit 221 may include a Bluetooth communication circuit. Additionally, the second communication circuit 221 may include a WiFi communication circuit. The second processor 229 may perform data communication with the wearable device 210 through the short-range wireless communication channel (e.g., a Bluetooth communication channel or a WiFi communication channel) established by the second communication circuit 211.

The second sensing circuit 222 may be configured to generate data used to detect a step and provide the generated data to the second processor 229. For example, the second sensing circuit 222 may include the inertia measurement unit (IMU) that is configured to include the acceleration sensor, the gyroscope, and the magnetometer.

A step detection module 240, a detection control module 250, a step number correction module 260, a reliability determination module 270, and a condition configuration module 280 may be configured as instructions, stored in a second memory 228, and executed by a second processor 229. The second processor 229 may store information indicating step information (e.g., the number of steps, distance, walking time zone) measured using the above-described modules 240 to 280 in the second memory 228.

The step detection module 240 may be configured to detect a step based on data received from the second sensing circuit 222. For example, the step detection module 240 may detect vibration patterns (e.g., vibration frequency, amplitude, and intensity) that satisfy predetermined step patterns from the data received from the first sensing circuit 212. The step detection module 240 may count steps whenever the vibration patterns are detected and store the counted number of steps in the second memory 228.

The detection control module 250 may be configured to control the step detection module 240. The step detection module 240 may perform or stop the step detection operation under the control of the detection control module 250. The step detection module 240 may continue the step detection but stop the counting operation based on the control of the detection control module 250. For example, the step detection module 240 may start counting from an N+1 step without counting steps detected from first to Nth.

According to an embodiment, the detection control module 250 may control the step detection module 240 to stop the step detection operation based on the reception of the first notification message (e.g., a message notifying that an exercise (bicycle, kick board, or motorcycle) other than walking and running is started) from the wearable device 210 through the second communication circuit 221. After the stop, the detection control module 250 may receive the third notification message (e.g., a message notifying that an exercise is ended) from the wearable device 210 through the second communication circuit 221. The detection control module 250 may control the step detection module 240 to resume the step detection operation based on the reception of the third notification message.

According to an embodiment, the second display 223 and/or the second input module 224 may be used as input devices that enables the user to determine the start and end of the bicycle exercise (or, kick board or motorcycle exercise).

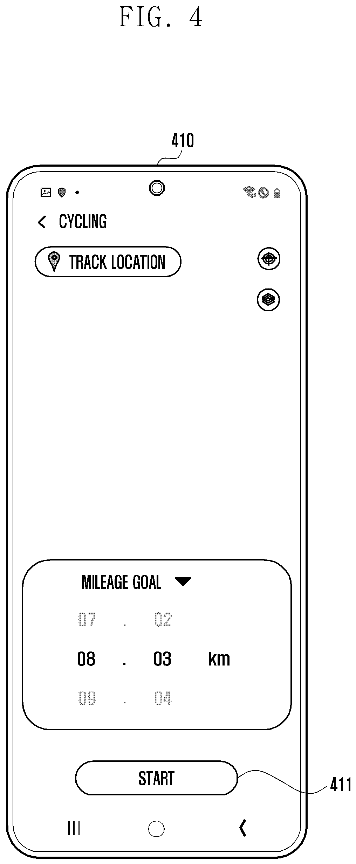

FIG. 4 illustrates an example of a UI screen displayed on the portable electronic device 220. Referring to FIG. 4, the second processor 229 may provide, to a user through the second display 223, a third UI screen 410 that enables the user to determine the start of the bicycle exercise. The detection exercise algorithm 250 may receive a user input indicating the start of the bicycle exercise through the third UI screen 410 displayed on the second display 223. For example, the detection control module 250 may receive a user's touch input for a display object 411 (e.g., an icon) indicated as 'start' on the third UI screen 410 from the second display 223. The second processor 229 may transmit a message indicating the start of the bicycle exercise to the wearable device 210 through the second communication circuit 221 in response to the reception of the user input indicating the start of the bicycle exercise through the second display 223. The bicycle exercise algorithm 230 may recognize the start of the bicycle exercise through the message received by the wearable device 210 The second processor 229 may not transmit the start message to the wearable device.

Referring to FIG. 2, the second processor 229 may provide, to a user through the second display 223, a fourth UI screen (not illustrated) that enables the user to determine the end of the bicycle exercise. The detection control module 250 may receive a user input indicating the end of the bicycle exercise through the fourth UI screen displayed on the second display 223. The second processor 229 may transmit a message indicating the end of the bicycle exercise to the wearable device 210 through the second communication circuit 221 in response to the reception of the user input indicating the end of the bicycle exercise through the second display 223. The bicycle exercise algorithm 230 may recognize the end of the bicycle exercise through the message received by the wearable device 210. The second processor 229 may not transmit the end message to the wearable device.

According to an embodiment, the portable electronic device 220 may provide, to a user through the second display 223, a UI screen that enables the user to determine the start and end of a kick board or motorcycle exercise.

The detection control module 250 may also receive a user input (e.g., a user input indicating the start of the bicycle, the kick board, or the motorcycle exercise) indicating the start of the exercise and a user input (e.g., a user input indicating the end of the bicycle, the kick board, or the motorcycle exercise) indicating the end of the exercise through the second input module 224 (e.g., a microphone or a physical button).

The detection control module 250 may control the step detection module 240 to stop the step detection operation based on the reception of the user input indicating the start of the exercise. The detection control module 250 may control the step detection module 240 to resume the step detection operation based on the reception of the user input indicating the end of the exercise.

As described above, the detection control module 250 may receive the user input from the second display 223 or the second input module 224 as an event that triggers stopping or resuming the step detection operation. The detection control module 250 may receive the notification message from the wearable device 210 as the event that triggers stopping or resuming the step detection operation. According to an embodiment, the detection control module 250 may maintain the stop of the step detection without resuming the step detection when the notification message to resume the step detection is received from the wearable device 210 while the step detection operation is stopped by the user input. According to an embodiment, the detection control module 250 may control the step detection module 240 to resume the step detection when the user input to resume the step detection is received from the second display 223 or the second input module 224 while the step detection operation is stopped by the notification message.

The step number correction module 260 may be configured to correct the number of steps (e.g., the accumulated number of steps measured and stored during a day) counted by the step detection module 240 and stored in the second memory 228 based on the exercise information (e.g., exercise information on the bicycle, the kick board, or the motorcycle) received from the wearable device 210 through the second communication circuit 221.

According to an embodiment, the wearable device 210 may include information indicating a first time point at which the exercise patterns (e.g., exercise patterns regarding the bicycle, the kick board, or the motorcycle) are first satisfied in the first notification message and transmit the first notification message to the portable electronic device 220. The step number correction module 260 may confirm the first time point in the first notification message. The step number correction module 260 may correct the number of steps by subtracting the number of steps counted by the step detection module 240 from the first time point to the second time point at which the first notification message is received from the number of steps stored in the second memory 228.

The reliability determination module 270 may be configured to receive the exercise information (e.g., exercise information on the bicycle, the kick board, or the motorcycle) from the wearable device 210. The reliability determination module 270 may be configured to determine reliability that indicates how likely the corresponding exercise is to be performed from the received exercise information. The reliability determination module 270 may be configured to determine the reliability as a high level in proportion to the exercise duration.

According to an embodiment, the reliability determination module 270 may receive the exercise information related to the exercise duration per unit time from the wearable device 210 through the second communication circuit 221. For example, the reliability determination module 270 may receive, from the wearable device 210, the first information including the exercise duration and the rest duration, the second information including a ratio of the exercise duration to the rest duration, or the third information including a multiple value that indicates how many times the exercise duration is greater than the rest duration. Based on the received information, the reliability determination module 270 may determine the reliability as one of the low level, the middle level, and the high level.

In the following embodiments, each operation may be performed sequentially, but is not necessarily performed sequentially. For example, the order of each operation may be changed, and at least two operations may be performed in parallel.

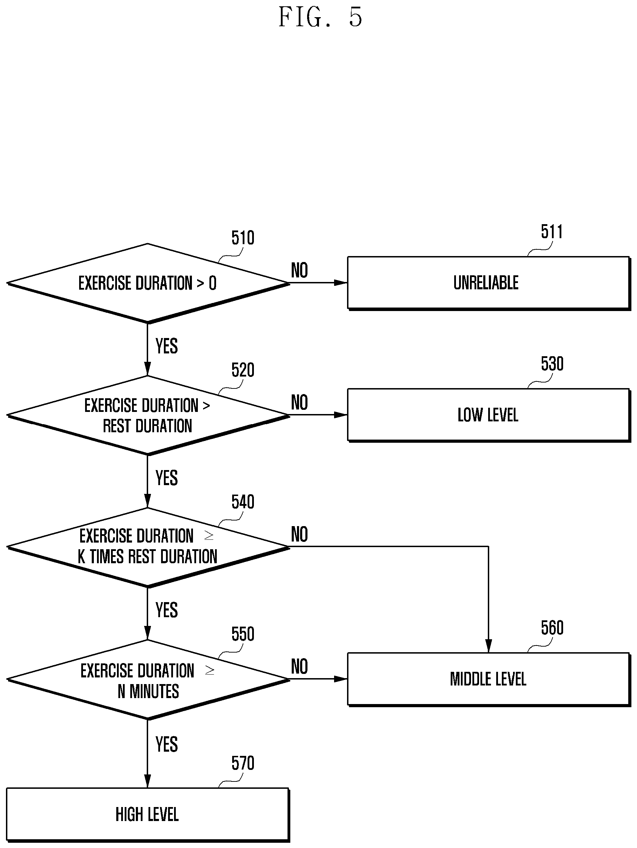

FIG. 5 is a flowchart for describing operations of a second processor 229 for determining reliability according to an embodiment. When the reliability determination module 270 is executed, the second processor 229 may perform the following operations based on the information (the first information, the second information, or the third information) received from the wearable device 210. In the description of FIG. 5, the exercise is specified as 'bicycle exercise', but is not limited thereto. That is, the operations of FIG. 5 may also be applied to other exercises (e.g., the kick board or the motorcycle).

It may be understood that operations 510 to 570 are performed by the second processor (e.g., the second processor 229 of FIG. 2) of the portable electronic device (e.g., the portable electronic device 220 of FIG. 2).

In operation 510, the second processor 229 may identify whether there is the exercise duration from the received information. If there is no exercise duration (exercise duration = 0), in operation 511, the second processor 229 may determine "no reliability (the user is unlikely to be riding a bicycle)".

When it is determined that there is the exercise duration, the second processor 229 may identify whether the exercise duration is longer than the rest duration from the received information in operation 520. When the exercise duration is not longer than the rest duration, in operation 530, the second processor 229 may determine the reliability as the low level.

When the exercise duration is longer than the rest duration, in operation 540, the second processor 229 may identify whether the exercise duration is k times (e.g., k is a number greater than 2) or longer the rest duration from the received information.

When the exercise duration is k times or longer the rest duration, in operation 550, the second processor 229 may identify whether the exercise duration is longer than or equal to a certain time (e.g., N minutes) from the received information.

When the exercise duration is shorter than k times the rest duration or the exercise duration is shorter than the certain time, in operation 560, the second processor 229 may determine the reliability as the middle level.

When the exercise duration is k times or more the rest duration or the exercise duration is longer than or equal to the certain time, in operation 570, the second processor 229 may determine the reliability as the high level.

The technical idea of the present disclosure is not limited to classifying reliability into four levels (no reliability, low, middle, high) as described above, and may be classified into five levels or less or five levels or more.

According to an embodiment, the detection control module 250 may control the step detection module 240 based on the reliability determined by the reliability determination module 270. The detection control module 250 may control the step detection module 240 to stop the step detection operation when the determined reliability is greater than or equal to a certain level (e.g., the high level of FIG. 5). The detection control module 250 may control the step detection module 240 to perform the step detection operation when the determined reliability is below a certain level (e.g., low level, middle level, or no reliability of FIG. 5).

The condition configuration module 280 may be configured to set a condition for starting the operation (hereinafter, "count") of counting steps based on the reliability determined by the reliability determination module 270. Here, the condition may include the number of steps or the characteristic values (e.g., the vibration frequency, the amplitude, the intensity) of the step pattern.

According to an embodiment, the condition configuration module 280 may control the step detection module 240 to start counting from an Nth step based on the determination that there is no reliability. For example, the step detection module 240 may count steps from an Nth step after a user takes his/her first step. The condition configuration module 280 may control the step detection module 240 to start the counting from an N+ath step (a is a positive integer) based on the determination that the reliability is the low level. The condition configuration module 280 may control the step detection module 240 to start the counting from an N+bth step (b is an integer greater than a) based on the determination that the reliability is the middle level.

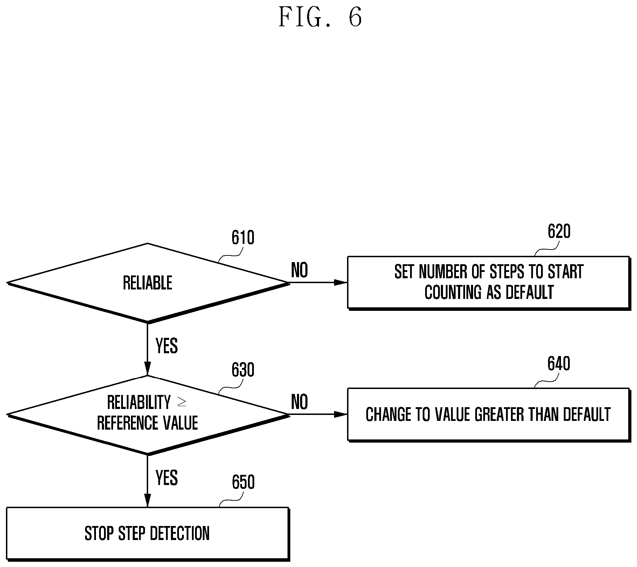

FIG. 6 is a flowchart for describing the operations of the second processor 229 for controlling step detection based on the reliability according to an embodiment. The detection control module 250 and the condition configuration module 280, may cause, when executed, the second processor 229 to perform the following operations based on the reliability determined by the reliability determination module 270.

It may be understood that operations 610 to 650 are performed by the second processor (e.g., the second processor 229 of FIG. 2) of the portable electronic device (e.g., the portable electronic device 220 of FIG. 2).

In operation 610, the second processor 229 may identify whether it has been determined that there is the reliability. When it is determined that there is no reliability, in operation 620, the second processor 229 may set the number of steps to start the counting as a default (e.g., N). The second processor 229 may start the counting from the step in the order corresponding to the value set to start the counting. For example, when the default is set to 5, the second processor 229 may start the counting from the fifth detected step without counting from the first detected step to the fourth detected step.

When it is determined that there is the reliability, in operation 630, the second processor 229 may identify whether the determined reliability is greater than or equal to a reference value. When the determined reliability is less than the reference value, in operation 640, the second processor 229 may change the number of steps to start the counting to a value greater than the default. When the determined reliability is greater than or equal to the reference value, in operation 650, the second processor 229 may stop the step detection operation using the second sensing circuit 222. For example, the reliability may be determined as the low level, the middle level, or the high level. When it is determined that the reliability is the low level, the second processor 229 may set a first value (e.g., N+a) greater than the default as a value to start the counting. When it is determined that the reliability is the middle level, the second processor 229 may set a second value (e.g., N+b) greater than the first value as a value to start the counting. When it is determined that the reliability is the high level, the second processor 229 may stop the step detection.

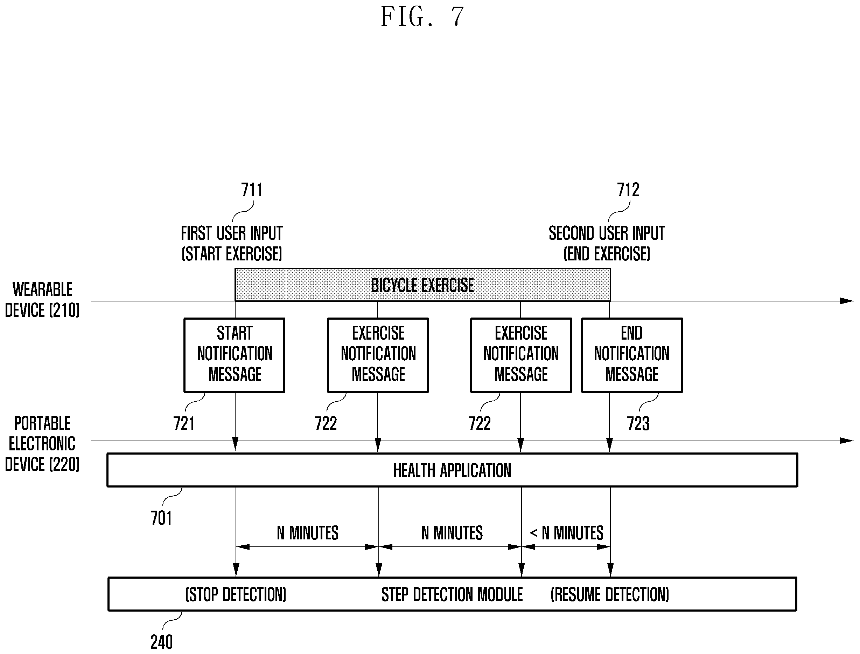

FIG. 7 is a diagram for describing operations of controlling step detection based on the user's determination on the start and end of the bicycle exercise in the system 200 of FIG. 2 according to an embodiment. In FIG. 7, a health application 701 (e.g., application 146 of FIG. 1) is configured to include a detection control module (e.g., the detection control module 250 of FIG. 2), and may be stored in a second memory (e.g., the second memory 228 of FIG. 2) of the portable electronic device 220 and executed by a second processor (e.g., the second processor 229 of FIG. 2). In the description of FIG. 7, the exercise is specified as 'bicycle exercise', but is not limited thereto. That is, the operations described with reference to FIG. 7 may also be applied to other exercises (e.g., the kick board or the motorcycle).

In the wearable device 210, a first processor (e.g., the first processor 219 of FIG. 2) may receive a first user input 711 indicating the start of the bicycle exercise from a user through a first display (e.g., the first display 213 of FIG. 2) or a first input module (e.g., the first input module 214 of FIG. 2). In response to receiving the first user input 711, the first processor 219 may transmit a start notification message 721 to the portable electronic device 220 through a first communication circuit (e.g., the first communication circuit 211 of FIG. 2). In the portable electronic device 220, the second processor 229 may receive the start notification message 721 through a second communication circuit (e.g., the second communication circuit 221 of FIG. 2). The health application 701 may transmit the start notification message 721 received by the portable electronic device 220 to a step detection module (e.g., the step detection module 240 of FIG. 2). The step detection module 240 may stop the step detection based on the reception of the start notification message 721. The health application 701 may store start time point information indicating that the bicycle exercise is started at the time point at which the start notification message 721 is received in the second memory 228.

In the wearable device 210, the first processor 219 may transmit, to the portable electronic device 220 through the first communication circuit 211, an exercise notification message 722 that notifies the portable electronic device 220 that the bicycle exercise is performing whenever a given period of time (N minutes) elapses after notifying the portable electronic device 220 of the start of the bicycle exercise. The health application 701 may transmit the exercise notification message 722 received by the portable electronic device 220 to the step detection module 240. The step detection module 240 may maintain the stop of the step detection based on the reception of the exercise notification message 722. The exercise notification message 722 may not be transmitted to the portable electronic device 220 even after a given period of time has elapsed due to the wireless connection being disconnected between the two devices 210 and 220. In this case, the step detection module 240 may resume the step detection.

Before a certain time (N minutes) has elapsed after the exercise notification message 722 is transmitted to the portable electronic device 220, the first processor 219 may receive the second user input 712 indicating the end of the bicycle exercise from the user through the first display 213 or the first input module 214. In response to receiving the second user input 712, the first processor 219 may transmit an end notification message 723 to the portable electronic device 220 through the first communication circuit 211. In the portable electronic device 220, the second processor 229 may receive the end notification message 723 through the second communication circuit 221. The health application 701 may transmit the end notification message 723 received by the portable electronic device 220 to the step detection module 240. The step detection module 240 may resume the step detection based on the reception of the end notification message 723. The health application 701 may store the end time point information indicating that the bicycle exercise is ended at the time point at which the end notification message 723 is received in the second memory 228.

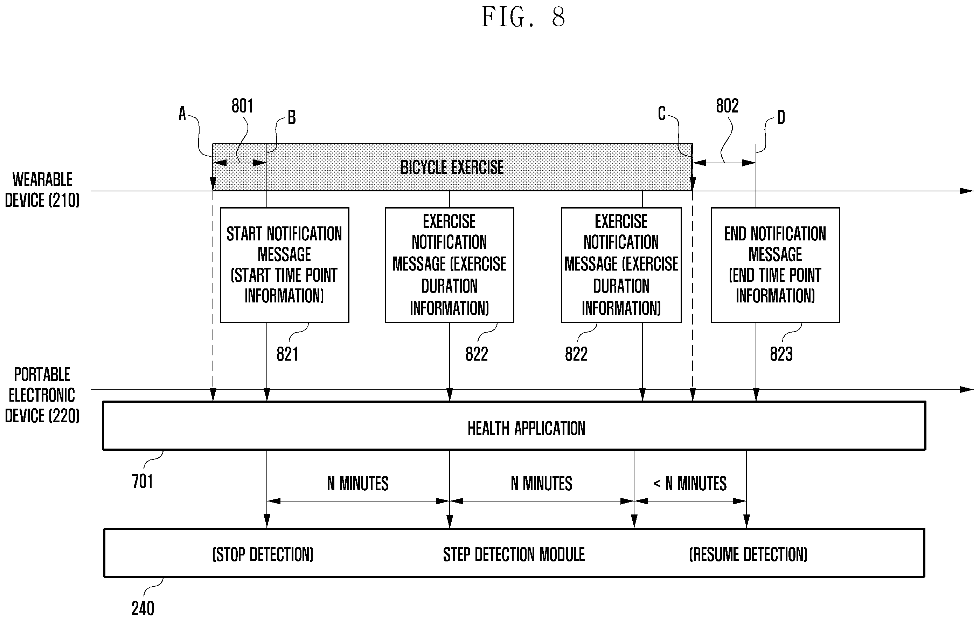

FIG. 8 is a diagram for describing the operations of controlling step detection based on the recognition of the start and end of the bicycle exercise in the system 200 of FIG. 2 according to an embodiment. In FIG. 8, the health application 701 is configured to include a detection control module (e.g., the detection control module 250 of FIG. 2), a step number correction module (e.g., the step number correction module 260 of FIG. 2), a reliability determination module (e.g., the reliability determination module 270 of FIG. 2), and a condition configuration module (e.g., the condition configuration module 280 of FIG. 2)), and may be stored in a second memory (e.g., the second memory 228 of FIG. 2) of the portable electronic device 220 and executed by a second processor (e.g., the second processor 229 of FIG. 2). In the description of FIG. 8, the exercise is specified as 'bicycle exercise', but is not limited thereto. That is, the operations described with reference to FIG. 8 may also be applied to other exercises (e.g., the kick board or the motorcycle).

In the wearable device 210, the first processor (e.g., the first processor 219 of FIG. 2) may recognize that physical quantities acquired through a first sensing circuit (e.g., the first sensing circuit 212 of FIG. 2) satisfy bicycle exercise patterns at the first time point A. Based on the fact that the physical quantities satisfying the bicycle exercise patterns are continuously acquired for a designated time 801 from a first time point A, the first processor 219 may transmit a start notification message 821 to the portable electronic device 220 through the first communication circuit (e.g., the first communication circuit 211 of FIG. 2) at a second time point B. The first processor 219 may include the start time point information on the first time point A in the start notification message 821 and transmit the start notification message 821 to the portable electronic device 220. In the portable electronic device 220, the second processor 229 may receive the start notification message 821 through the second communication circuit (e.g., the second communication circuit 221 of FIG. 2) at the second time point B. The health application 701 may transmit the start notification message 821 received by the portable electronic device 220 to the step detection module (e.g., the step detection module 240 of FIG. 2). The step detection module 240 may stop the step detection based on the reception of the start notification message 821. The health application 701 may identify the first time point A in the start notification message 821, and the step detection module 240 may correct the number of steps by subtracting the number of steps counted from the first time point A to the second time point B from the number of steps (e.g., the number of daily steps) stored in the second memory 228. The health application 701 may store the start time point information indicating that the bicycle exercise is started at the first time point A in the second memory 228.

In the wearable device 210, the first processor 219 may transmit, to the portable electronic device 220 through the first communication circuit 211, an exercise notification message 822 that notifies the portable electronic device 220 that the bicycle exercise is performing whenever the given period of time (N minutes) elapses after notifying the portable electronic device 220 of the start of the bicycle exercise. The first processor 219 may include the exercise duration information in the start notification message 821 and transmit the start notification message 821 to the portable electronic device 220. For example, the first processor 219 may include the first information indicating the exercise duration and the rest duration per unit time (N minutes), the second information indicating the ratio of the exercise duration to the rest duration, or the information that indicates how many times the exercise duration is greater than the rest duration in the exercise notification message 822, and transmit the exercise notification message 822 to the portable electronic device 220.

The health application 701 may determine whether to maintain the stop of the step detection based on the exercise duration information included in the exercise notification message 822. According to an embodiment, the health application 701 may determine the reliability of the bicycle exercise as one of no reliability, the low level, the middle level, and the high level, as exemplarily described with reference to FIG. 5, based on the exercise duration information included in the exercise notification message 822. When the determined reliability is the high level, the health application 701 may transmit the exercise notification message 822 to the step detection module 240 to maintain the stop of the step detection. When the determined reliability is the low level or the middle level, the health application 701 may control the step detection module 240 to change the number of steps to start the counting to the value greater than the default (e.g., operation 640) and resume the step detection.

The exercise notification message 822 may not be transmitted to the portable electronic device 220 even after the given period of time has elapsed due to the wireless connection being disconnected between the two devices 210 and 220. In this case, the step detection module 240 may resume the step detection.

Before a certain time (N minutes) has elapsed after the exercise notification message 822 is transmitted to the portable electronic device 220, the first processor 219 in the wearable device 210 may recognize that the physical quantities acquired through the first sensing circuit 212 do not satisfy the bicycle exercise patterns at the third time point C. Based on the fact that the physical quantities not satisfying the bicycle exercise patterns are continuously acquired for the designated time 802 from the third time point C, the first processor 219 may transmit an end notification message 823 to the portable electronic device 220 through the first communication circuit 211 at a fourth time point D. The first processor 219 may include the end time point information on the third time point C in the end notification message 823 and transmit the end notification message 823 to the portable electronic device 220. The second processor 229 in the portable electronic device 220 may receive the end notification message 823 through the second communication circuit 221 at the fourth time point D. The health application 701 may transmit the end notification message 823 received by the portable electronic device 220 to the step detection module 240. The step detection module 240 may resume the step detection based on the reception of the end notification message 823. The health application 701 may identify the third time point C in the end notification message 823 and store the end time point information indicating that the bicycle exercise is ended at the third time point C in the second memory 228.

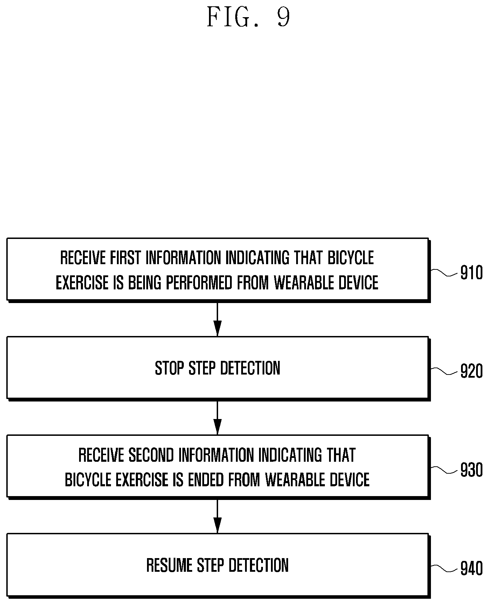

FIG. 9 is a flowchart for describing operations performed by the second processor 229 of the portable electronic device 220 based on the information received from the wearable device 210 according to an embodiment. In the description of FIG. 9, the exercise is specified as 'bicycle exercise', but is not limited thereto. That is, the operations described with reference to FIG. 9 may also be applied to other exercises (e.g., the kick board or the motorcycle).

It may be understood that operations 910 to 940 are performed by the second processor (e.g., the second processor 229 of FIG. 2) of the portable electronic device (e.g., the portable electronic device 220 of FIG. 2).

In operation 910, the second processor 229 may receive the first information (e.g., the start notification message 721 or 821) that indicates that the bicycle exercise is performing from the wearable device 210 through the second communication circuit 221.

In operation 920, the second processor 229 may stop the step detection operation using the second sensing circuit 222 based on the reception of the first information.

While the step detection operation is stopped, in operation 930, the second processor 229 may receive the second information (e.g., the end notification message 723 or 823) that indicates that the bicycle exercise is ended from the wearable device 210 through the second communication circuit 221.

In operation 940, the second processor 229 may resume the step detection based on the reception of the second information.

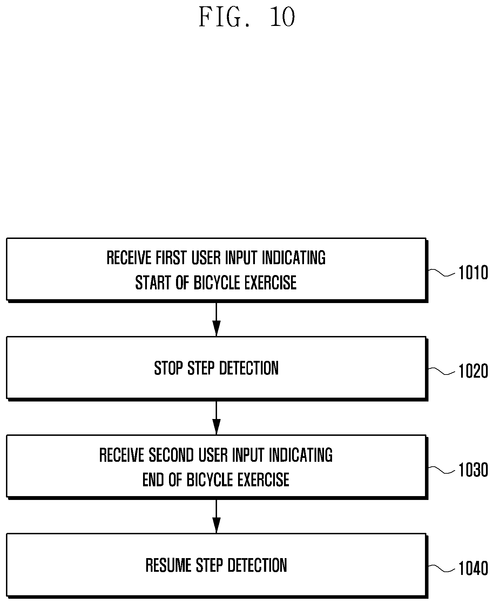

FIG. 10 is a flowchart for describing the operations performed by the second processor 229 of the portable electronic device 220 based on the user input according to an embodiment. In the description of FIG. 10, the exercise is specified as 'bicycle exercise', but is not limited thereto. That is, the operations described with reference to FIG. 10 may also be applied to other exercises (e.g., the kick board or the motorcycle).

It may be understood that operations 1010 to 1040 are performed by the second processor (e.g., the second processor 229 of FIG. 2) of the portable electronic device (e.g., the portable electronic device 220 of FIG. 2).

In operation 1010, the second processor 229 may receive the first user input indicating the start of the bicycle exercise from the second display 223 or the second input module 224.

In operation 1020, the second processor 229 may stop the step detection based on the reception of the first user input.

While the step detection operation is stopped, in operation 1030, the second processor 229 may receive the second user input indicating the end of the bicycle exercise from the second display 223 or the second input module 224.

In operation 1040, the second processor 229 may resume the step detection based on the reception of the second user input.

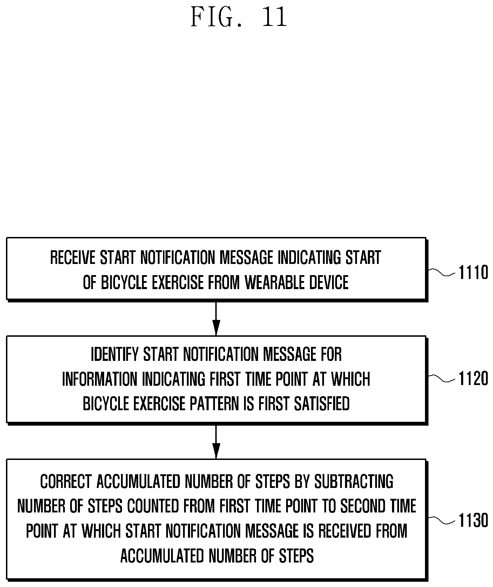

FIG. 11 is a flowchart for describing the operations performed based on the information received from the wearable device 210 according to an embodiment. In the description of FIG. 11, the exercise is specified as 'bicycle exercise', but is not limited thereto. That is, the operations described with reference to FIG. 11 may also be applied to other exercises (e.g., the kick board or the motorcycle).

It may be understood that operations 1110 to 1130 are performed by the second processor (e.g., the second processor 229 of FIG. 2) of the portable electronic device (e.g., the portable electronic device 220 of FIG. 2).

In operation 1110, the second processor 229 may receive the start notification message (e.g., the start notification message 821) indicating the start of the bicycle exercise from the wearable device 210 through the second communication circuit 221.

In operation 1120, the second processor 229 may identify the information indicating the first time point at which the bicycle exercise patterns are first satisfied (e.g., as the time point A in FIG. 8, the time point at which the physical quantities acquired from the wearable device 210 are first recognized to satisfy the bicycle exercise patterns) in the start notification message.

In operation 1130, the second processor 229 may correct the accumulated number of steps by subtracting the number of steps counted from the first time point to the second time point at which the start notification message is received from the accumulated number of steps (e.g., the number of daily steps) stored in the second memory 228.

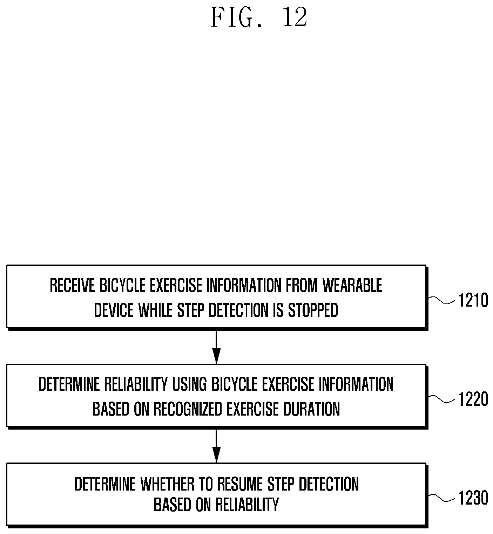

FIG. 12 is a flowchart for describing the operations performed based on the information received from the wearable device 210 according to an embodiment. In the description of FIG. 12, the exercise is specified as 'bicycle exercise', but is not limited thereto. That is, the operations described with reference to FIG. 12 may also be applied to other exercises (e.g., the kick board or the motorcycle).

It may be understood that operations 1210 to 1230 are performed by the second processor (e.g., the second processor 229 of FIG. 2) of the portable electronic device (e.g., the portable electronic device 220 of FIG. 2).

While the step detection is stopped, in operation 1210, the second processor 229 may receive the bicycle exercise information (e.g., exercise notification message 722 or 822) related to the time when the bicycle exercise was performed per unit time from the wearable device 210 through the second communication circuit 221.

In operation 1220, the second processor 229 may determine the reliability based on the exercise duration recognized using the bicycle exercise information. For example, as described with reference to FIG. 5, the second processor 229 may determine the reliability as one of "no", the low level, the middle level, and the high level based on the exercise duration.

In operation 1230, the second processor 229 may determine whether to resume the step detection based on the determined reliability. For example, when it is determined that the reliability is the high level, the second processor 229 may maintain the stop of the step detection. When it is determined that there is no reliability or when it is determined that the reliability is the middle level or the low level, the second processor 229 may resume the step detection. When it is determined that there is no reliability, the second processor 229 may set the number of steps to start the counting as the default (N numbers). When it is determined that the reliability is the middle level or the low level, the second processor 229 may set the number of steps to start the counting as the value greater than the default. For example, when it is determined that the reliability is the low level, the second processor 229 may set the first value (e.g., N+a) greater than the default (N numbers) as the value to start the counting. When it is determined that the reliability is the middle level, the second processor 229 may set the second value (e.g., N+b) greater than the first value as the value to start the counting.

In the above description, the prefixes "first," "second," "third," etc., are only used to distinguish between the same names and do not have any special meaning in themselves, such as importance or order.

According to an embodiment, the portable electronic device (e.g., portable electronic device 220) includes: a wireless communication circuit; a sensing circuit; a processor connected to the wireless communication circuit and the sensing circuit; and a memory operatively connected to the processor. The memory may store instructions which cause, when executed, the processor to receive first information indicating that an exercise other than walking and running is being performed, from a wearable device (e.g., wearable device 210) through the wireless communication circuit. The instructions may cause the processor to stop the step detection operation using the sensing circuit based on the reception of the first information. The instructions may cause the processor to receive the second information indicating that the exercise is ended, from the wearable device through the wireless communication circuit, while the detection operation is stopped. The instructions may cause the processor to resume the detection operation based on the reception of the second information.

The information indicating the first time point along with the first information may be received by the portable electronic device. The instructions may cause the processor to subtract the number of steps counted from the first time point to the second time point at which the first information is received from the accumulated number of steps.

The instructions may cause the processor to receive the exercise information related to the time when an exercise is performed per unit time from the wearable device through the wireless communication circuit, while the detection operation is stopped. The instructions may cause the processor to determine whether to resume the detection operation based on the exercise information.

The instructions may cause the processor to determine the reliability that indicates how likely the exercise is to be performed based on the recognized exercise duration using the exercise information. The instructions may cause the processor to resume the detection operation based on the determination that the reliability is greater than or equal to the designated reference value. The instructions may cause the processor to maintain the stop of the detection operation based on the determination that the reliability is lower than the reference value.

The instructions may cause the processor to determine the reliability that indicates how likely the exercise is to be performed based on the recognized exercise duration using the exercise information. The instructions may cause the processor to set the number of steps to start the counting as the default based on the determination that there is no reliability. The instructions may cause the processor to set the number of steps to start the counting as the first value greater than the default based on the determination that the reliability is the low level. The instructions may cause the processor to set the number of steps to start the counting as the second value greater than the first value based on the determination that the reliability is the middle level. The instructions may cause the processor to maintain the stop of the detection operation based on the determination that the reliability is the high level.

The instructions may cause the processor to determine that there is no reliability based on no recognized exercise duration. The instructions may cause the processor to determine the reliability as the low level based on the fact that the recognized exercise duration is shorter than the rest duration. The instructions may cause the processor to determine the reliability as the middle level based on the fact that the recognized exercise duration is longer than the rest duration and shorter than k times the rest duration; The instructions may cause the processor to determine the reliability as the middle level based on the fact that the recognized exercise duration is k times or longer the rest duration and shorter than N minutes. The instructions may cause the processor to determine the reliability as the high level based on the recognized exercise duration being longer than or equal to k times the rest duration and longer than or equal to N minutes.

The instructions may cause the processor to receive, as the exercise information, from the wearable device through the wireless communication circuit, the first information including the exercise duration and the rest duration, the second information including the ratio of the exercise duration to the rest duration, or the third information including the multiple value indicating how much longer the exercise duration is compared to the rest duration.

According to an embodiment, a portable electronic device (e.g., the portable electronic device 220) includes: a touch-sensitive display; an input device; a sensing circuit; a processor connected to the display, the input device, and the sensing circuit; and a memory operatively connected to the processor. The memory may store instructions which cause, when executed, the processor to receive first information indicating that an exercise other than walking and running is being performed, from the display or the input device. The instructions may cause the processor to stop the step detection operation using the sensing circuit based on the reception of the first information. The instructions may cause the processor to receive second information indicating that the exercise is ended, from the display or the input device, while the detection operation is stopped. The instructions may cause the processor to resume the detection operation based on the reception of the second information.

According to an embodiment, a method of operating a portable electronic device (e.g., the portable electronic device 220) may include receiving, from a wearable device (e.g., wearable device 210) through a wireless communication circuit of the portable electronic device, first information indicating that an exercise other than walking and running is performing. The method may include stopping a step detection operation using the sensing circuit of the portable electronic device based on the reception of the first information. The method may include receiving second information indicating that the exercise is ended, from the wearable device through the wireless communication circuit, while the detection operation is stopped. The method may include resuming the detection operation based on the reception of the second information.

The information indicating the first time point along with the first information may be received by the portable electronic device. The method may include subtracting the number of steps counted from the first time point to a second time point at which the first information is received from the accumulated number of steps.

The method may include receiving the exercise information related to the time when an exercise is performed per unit time from the wearable device through the wireless communication circuit, while the detection operation is stopped. The method may include determining whether to resume the detection operation based on the exercise information.

The determining of whether to resume the detection operation may include: determining reliability that indicates how likely the exercise is to be performed based on a recognized exercise duration using the exercise information; resuming the detection operation based on the reliability that is determined to be greater than or equal to a designated reference value; and maintaining the stop of the detection operation based on the reliability that is determined to be lower than the reference value.

The determining of whether to resume the detection operation may further include: determining reliability that indicates how likely the exercise is to be performed based on a recognized exercise duration using the exercise information; setting the number of steps to start counting as a default based on the determination that there is no reliability; setting the number of steps to start the counting as a first value greater than the default based on the determination that the reliability is a low level; setting the number of steps to start the counting as a second value greater than the first value based on the determination that the reliability is a middle level; and maintaining the stop of the detection operation based on the determination that the reliability is a high level.

The determining of the reliability may include: determining that there is no reliability based on no recognized exercise duration;
determining the reliability as the low level based on the recognized exercise duration being shorter than a rest duration; determining the reliability as the middle level based on the recognized exercise duration being longer than the rest duration and shorter than k times the rest duration; determining the reliability as the middle level based on the recognized exercise duration being k times or longer the rest duration and shorter than N minutes; and determining the reliability as the high level based on the recognized exercise duration being k times or longer the rest duration and longer than or equal to N minutes.

The exercise information may include first information including an exercise duration and a rest duration, second information including a ratio of the exercise duration to the rest duration, or third information including a multiple value that indicates how many times the exercise duration is greater than the rest duration.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A portable electronic device, comprising:
a wireless communication circuit;
a sensing circuit;
a processor connected to the wireless communication circuit and the sensing circuit; and
a memory operatively connected to the processor,
wherein the memory stores instructions which cause, when executed, the processor to:
receive first information indicating that an exercise other than walking and running is being performed, from a wearable device through the wireless communication circuit;
stop a step detection operation using the sensing circuit based on the reception of the first information;
receive second information indicating that the exercise is ended, from the wearable device through the wireless communication circuit, while the detection operation is stopped; and
resume the detection operation based on the reception of the second information.

2. The portable electronic device of claim 1, wherein information indicating a first time point is received by the portable electronic device along with the first information, and
the instructions cause the processor to subtract the number of steps counted from the first time point to a second time point at which the first information is received from the accumulated number of steps.

3. The portable electronic device of claim 1, wherein the instructions cause the processor to receive exercise information related to a time when an exercise is performed during a predetermined duration of time from the wearable device through the wireless communication circuit, while the detection operation is stopped, and
determine whether to resume the detection operation based on the exercise information.

4. The portable electronic device of claim 3, wherein the instructions cause the processor to determine reliability that indicates how likely the exercise is to be performed based on a recognized exercise duration using the exercise information,
resume the detection operation based on the reliability that is determined to be greater than or equal to a designated reference value, and
maintain the stop of the detection operation based on the reliability that is determined to be lower than the reference value.

5. The portable electronic device of claim 3, wherein the instructions cause the processor to determine reliability that indicates how likely the exercise is to be performed based on a recognized exercise duration using the exercise information,
set the number of steps to start counting as a default based on the determination that there is no reliability,
set the number of steps to start the counting as a first value greater than the default based on the determination that the reliability is a low level,
set the number of steps to start the counting as a second value greater than the first value based on the determination that the reliability is a middle level, and
maintain the stop of the detection operation based on the determination that the reliability is a high level.

6. The portable electronic device of claim 5, wherein the instructions cause the processor to determine that there is no reliability based on no recognized exercise duration,
determine the reliability as the low level based on the recognized exercise duration being shorter than a rest duration,
determine the reliability as the middle level based on the recognized exercise duration being longer than the rest duration and shorter than k times the rest duration,
determine the reliability as the middle level based on the recognized exercise duration being k times or longer the rest duration and shorter than N minutes, and
determine the reliability as the high level based on the recognized exercise duration being k times or longer the rest duration and longer than or equal to N minutes.

7. The portable electronic device of claim 3, wherein the instructions cause the processor to receive, as the exercise information, from the wearable device through the wireless communication circuit, first information including an exercise duration and a rest duration, second information including a ratio of the exercise duration to the rest duration, or third information including a multiple value that indicates how many times the exercise duration is greater than the rest duration.

8. A portable electronic device, comprising:
a touch-sensitive display;
an input device;
a sensing circuit;
a processor connected to the display, the input device, and the sensing circuit; and
a memory operatively connected to the processor,
wherein the memory stores instructions which cause, when executed, the processor to:
receive first information indicating that an exercise other than walking and running is being performed, from the display or the input device;
stop a step detection operation using the sensing circuit based on the reception of the first information;
receive second information indicating that the exercise is ended, from the display or the input device, while the detection operation is stopped; and
resume the detection operation based on the reception of the second information.

9. A method of operating a portable electronic device, comprising:
receiving first information indicating that an exercise other than walking and running is being performed, from a wearable device through a wireless communication circuit of the portable electronic device;
stopping a step detection operation using the sensing circuit of the portable electronic device based on the reception of the first information;
receiving second information indicating that the exercise is ended, from the wearable device through the wireless communication circuit, while the detection operation is stopped; and
resuming the detection operation based on the reception of the second information.

10. The method of claim 9, wherein information indicating a first time point is received by the portable electronic device along with the first information, and
the method further comprises:
subtracting the number of steps counted from the first time point to a second time point at which the first information is received from the accumulated number of steps.

11. The method of claim 9, further comprising:
receiving exercise information related to a time when an exercise is performed during a predetermined duration of time from the wearable device through the wireless communication circuit, while the detection operation is stopped; and
determining whether to resume the detection operation based on the exercise information.

12. The method of claim 11, wherein the determining of whether to resume the detection operation includes:
determining reliability that indicates how likely the exercise is to be performed based on a recognized exercise duration using the exercise information;
resuming the detection operation based on the reliability that is determined to be greater than or equal to a designated reference value; and
maintaining the stop of the detection operation based on the reliability that is determined to be lower than the reference value.

13. The method of claim 11, wherein the determining of whether to resume the detection operation further includes:
determining reliability that indicates how likely the exercise is to be performed based on a recognized exercise duration using the exercise information;
setting the number of steps to start counting as a default based on the determination that there is no reliability;
setting the number of steps to start the counting as a first value greater than the default based on the determination that the reliability is a low level;
setting the number of steps to start the counting as a second value greater than the first value based on the determination that the reliability is a middle level; and
maintaining the stop of the detection operation based on the determination that the reliability is a high level.

14. The method of claim 13, wherein the determining of the reliability includes:
determining that there is no reliability based on no recognized exercise duration;
determining the reliability as the low level based on the recognized exercise duration being shorter than a rest duration;
determining the reliability as the middle level based on the recognized exercise duration being longer than the rest duration and shorter than k times the rest duration;
determining the reliability as the middle level based on the recognized exercise duration being k times or longer the rest duration and shorter than N minutes; and
determining the reliability as the high level based on the recognized exercise duration being k times or longer the rest duration and longer than or equal to N minutes.

15. The method of claim 11, wherein the exercise information includes first information including an exercise duration and a rest duration, second information including a ratio of the exercise duration to the rest duration, or third information including a multiple value that indicates how many times the exercise duration is greater than the rest duration.
